# EUROPEAN PATENT APPLICATION

(11) **EP 1 872 745 A2**
(43) Date of publication of application: **02.01.2008**
(21) Application number: 07111027.4
(22) Date of filing: 26.06.2007
(51) Int. Cl.: A61F 2/36, A61F 2/46

(54) **Femoral head resurfacing**

(30) Priority: 30.06.2006 US 478870
(71) Applicant: Howmedica Osteonics Corp., Mahwah, NJ 07430 (US)
(72) Inventor: Ledger, Robert, RIVER VALE, NJ 07675 (US); Tulkis, Peter, PARAMUS, NJ 07652 (US); Wang, Aiguo, WAYNE, NJ 07470 (US); Servidio, Damon, TOWACO, NJ 07082 (US)
(74) Representative: Le Forestier, Eric

(57) **Abstract**

A hip resurfacing femoral prosthesis has a partial ball component (20) having an outer surface (22) shaped to conform to an acetabular socket and has a mating sleeve component (10) with an internal bore (14) adapted to receive a femoral head (7). The head (7) has been shaped (71) and dimensioned to engage the bore (14) and is retained by bone ingrowth, an interference fit or by bone cement. The ball component (20) and sleeve (10) axes (E,F) may be offset to reposition the outer surface (22).

## Description

### BACKGROUND OF THE INVENTION

The present invention relates generally to systems, kits and methods for joint replacement using multiple components. In one embodiment, the present invention includes as components a ball component and a sleeve component for adapting the ball component to a prepared femoral head.

Artificial joint prostheses are widely used today, restoring joint mobility to patients affected by a variety of conditions, including degeneration of the joint and bone structure. Typically, the failed bone structure is replaced with an orthopedic implant that mimics, as closely as possible, the structure of the natural bone and performs its functions. The satisfactory performance of these implants can be affected not only by the design of the component itself, but also by the surgical positioning of the implanted component and the long-term fixation of the implant. Improper placement or positioning of the implant can adversely affect the goal of satisfactorily restoring the clinical bio-mechanics of the joint as well as impairing adequate fixation of the component when implanted.

Orthopedic implants are constructed from materials that are stable in biological environments and withstand physical stress with minimal or controlled deformation. Such materials must possess strength, resistance to corrosion, biocompatibility, and good wear properties. Also, the implants include various interacting parts, which undergo repeated long-term physical stress inside the body.

For these reasons, among others, the bone/implant interface and the connection between various parts of the implant must be durable and resistant to breakdown. This is especially important since installation of an orthopedic implant often involves an extensive and difficult medical procedure, and therefore replacement or revision of the installed implant is typically difficult and traumatic.

The requirements for the useful life of the implant continue to grow with the increase in human life expectancy.

Also, as implants improve, younger patients are considered as implant candidates. It is therefore desirable to develop implants that, while durable in their own right, minimize the difficulty of replacement or revision surgery should the implant eventually fail.

The strength and longevity of implants in large part depend on the bone/implant interface. Various methods of connection are known in the art. For example, a hip joint is a ball-in-socket joint, and includes a rounded femoral head and a cup-like socket (acetabular cup) located in the pelvis. The surfaces of the rounded femoral head and the acetabular cup continually abrade each other as a person walks. The abrasion, along with normal loading, creates stress on the hip joint and adjacent bones. If the femoral head or the acetabular cup is replaced with an implant, this stress must be well tolerated by the implant's bearing surfaces to prevent implant failure.

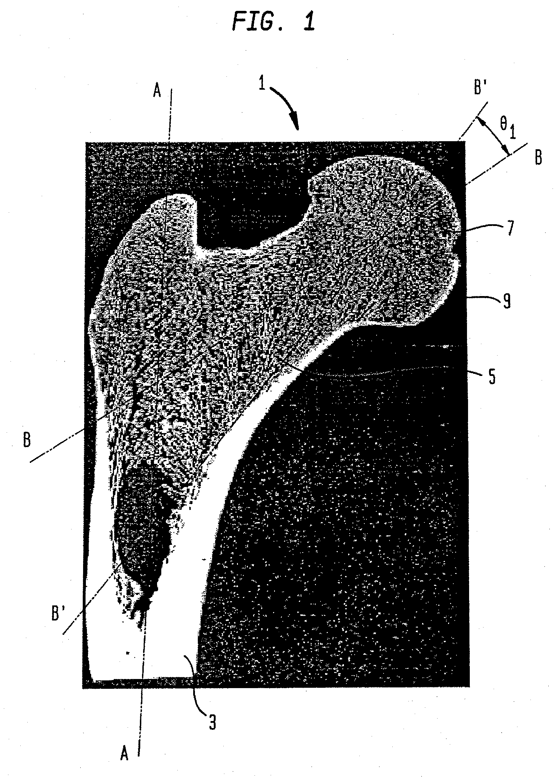

Depending on the type of bone, the location of the bone within the body and individual characteristics, bone has a wide variation in mechanical characteristics. Bone is generally categorized as trabecular or cancellous bone, which is porous and has an open cancellated structure, and cortical bone, which is dense. Considering the femoral bone of the hip joint, FIG. 1 shows the proximal portion of a femur 1 with the upper portion of the shaft 3, a neck 5 and a head 7. An axis A-A is aligned with the shaft 3 and an axis B-B is aligned with the neck 5. The shaft 3 is primarily composed of cortical bone while the neck 5 and head 7 are primarily composed of trabecular bone with cortical bone at the surface.

Implantable joint prostheses have long been used to provide an artificial hip. When the prosthesis is situated in this position, significant forces such as axial, bending, and rotational forces are imparted to the device. Conventional total hip replacements use an intramedullary stem as part of the femoral prosthesis. The stem passes into the marrow cavity of the femoral shaft. These stem type prostheses are very successful but when they fail the stem can create considerable damage inside the bone. The implant can move about inside the bone causing the intramedullary cavity to be damaged. Because a stiff stem transmits the forces more directly into the femoral shaft, such implants have the further disadvantage that they can weaken the surrounding bone proximal to the hip joint due to stress shielding.

Early designs of femoral prostheses for artificial hips relied primarily on cemented fixation. These cements, such as polymethylmethacrylate, were used to anchor the component within the medullary canal by acting as a grouting agent between the component and the endosteal (inner) surface of the bone. While this method of fixation by cement provides immediate fixation and resistance to the forces encountered, and allows the surgeon to effectively position the device before the cement sets, it is not without problems. Over time, the mechanical properties and the adhesive properties of the bone cement degrade; eventually the forces overcome the cement and cause the components to become loose due to a failure at the cement/bone or cement/stem interface. Alternative approaches to address the issue of cement failure include both biological ingrowth and press-fit type stems.

Stems designed for biological ingrowth typically rely on the bone itself to grow into a specially prepared surface of the component, resulting in firmly anchoring the implant within the medullary canal. A shortfall of this approach is that, in contrast to components that utilize cement fixation, surfaces designed for biological ingrowth do not provide for immediate fixation because it takes time for the bone to grow into the specially prepared surface. Press-fit stems precisely engineered to fit within a surgically prepared medullary canal may or may not have specially prepared surfaces and typically rely on an interference fit of some portion of the component within the medullary canal of the bone to achieve stable fixation.

The need often arises to replace at least a portion of a hip implant. Prior art designs often require the entire implant to be replaced even if only a portion of the implant fails. Similarly, the entire implant may have to be replaced if the implant is intact but certain conditions surrounding the implant have changed. This is often due to the implant suffering from a decrease in support from the adjacent bone from stress shielding or other negative effects of the implant on surrounding bone.

Surgeons have sought a more conservative device than an implant using an intramedullary stem as part of the femoral prosthesis. There have been a number of attempts at implants using short stems or femoral caps without stems and requiring less extensive surgery. This type of prosthesis is generally known as a hip resurfacing prosthesis as opposed to a total hip prosthesis. In the mid-1940's Judet in France designed a prosthesis whereby the majority of the femoral head was removed and a replacement device was fitted with a peg or nail which passed a short way down the femoral neck. Small movement of the device against the bone caused friction of the bone and the bending loads on the peg often caused them to break out underneath the bony femoral neck. In the mid-1970's, double cup type arthroplasty was tried. There were several designs: Wagner in Germany, an Italian Group, Imperial College London and the Tharies design from Amstutz in California. These all removed a fair proportion of the femoral bearing surface by turning it down to a cylindrical form or hemispherical form. A metal shell was then fixed with bone cement on the remaining bony peg. The acetabular cup was conventional. Unlike normal total hips, however, which have standard femoral head sizes in the range of 22-32 mm, these double cup arthroplasties needed to have large bearing surface diameters closer to the original hip, typically in a range from 40-60 mm. These latter double cup designs commonly failed either by a crack progressing around the bone cement between the prosthetic femoral shell and the bone or by a fracture of the bone across from one side of the prosthetic femoral component rim to the other.

Current approaches to femoral head resurfacing can be traced back to Amstutz in U.S. Patent 4,123,806. In the '806 patent, a hemispherical cap is cemented to a prepared femoral head while preserving a substantial portion of the femoral head. In U.S. Patent 6,156,069, Amstutz shows a femoral head resurfacing implant having a stem. A similar femoral head resurfacing technique called Birmingham Hip Resurfacing has been developed by McMinn in the United Kingdom. A modular approach to a femoral hip resurfacing is shown in U.S. Patent 4,846,841 to Oh. In this approach, a frustro-conical cap is press-fit to a prepared femoral head. A ball component is then attached to and retained by the cap using a Morse taper fit. A similar approach is shown in U.S. Patent 5,258,033 to Lawes and Ling, which shows a ball component cemented either directly to a prepared head or additionally retained by a press-fit with a frustro-conical cap.

All of these more modern hip resurfacing approaches require that the femoral head be prepared to provide a properly oriented and shaped bone interface for the implant by shaping the head. The outer prepared bone interface with the implant is usually symmetrical around an axis passing through the central region of the femoral neck and is typically cylindrical or conical but may be a more complex solid of revolution. The proximal portion of the prepared head can be a flat surface, tapered, domed, chamfered, or any combination of these features and is usually performed as a separate resection following preparation of the outer interface surface. If a stem is used, it may be cylindrical, tapered or a more complex solid of revolution and is typically short compared to a conventional intramedullary stem. The portion of the bone that hosts the prosthesis must be shaped so that it matches the shape of the prosthesis. The size and shape of the bone may fit exactly the shape and size of the prosthesis or may provide room for cementing to take place or have an excess of bone in a region to allow press-fit fixation, depending on the preferred fixation method.

Because the desired bone shape of the outer implant interface is symmetrical around an axis, a guide wire introduced into the femoral head is typically used to establish the tooling landmark for the various measuring and cutting tools used in the preparation process by providing an axis of revolution. Based on pre-operative planning, the surgeon initially places the guide wire, either freehand or using measurement and guidance tools based on various anatomical reference points on the femur. In order to place the pin, the pin is impacted or inserted in the proximal surface of the femoral head directed toward the greater trochanter and approximately down the mid-lateral axis of the femoral neck. A gauge having an extended stylus that allows measurement of the position of the pin with respect to the neck is then typically used to make a preliminary check of the pin position. By revolving the gauge, the surgeon can evaluate the position of the pin to ensure that the femoral neck will not be undercut when the cutting tool is revolved around the pin. The surgeon also uses the gauge to evaluate the support the prepared femoral head will provide to the implant. If the surgeon is satisfied that the pin position meets these criteria, the guide wire is used to establish the axis of revolution for the shaping cutter or reamer to prepare the head to receive the implant. If a stem cavity is required, a cannulated drill or reamer is centered on the guide pin to create the cavity after creating the outer surface of the prepared head.

### SUMMARY OF THE INVENTION

It is an object of the present invention to provide a more successful surface replacement of the femoral portion of a total hip replacement by improvements to a stemless, modular approach to femoral hip resurfacing.

According to an aspect of the present invention, a total hip replacement femoral component has an outer ball component sized to conform to an acetabular socket. The ball component is hemispherical and has an internal bore adapted to receive the outer surface of a sleeve. The bore and sleeve outer surface have mating surfaces typically in the shape of a truncated cone to create a taper lock type fit, but may also incorporate anti-rotational or indexing features such as a tapered spline, tapered square or a keyway and key. The inner surface of the sleeve is shaped and dimensioned to mate with a prepared femoral head. The sleeve and prepared head may also incorporate anti-rotational or indexing features. The sleeve receives the head and is retained by various known methods including bone ingrowth, an interference fit or by using bone cement.

It is another aspect of the invention to provide ball and sleeve components with altered geometries to allow variation in the orientation of the ball component with respect to the axis defined by the femoral head and neck and to provide a system of location features to facilitate adjusting the ball component orientation during surgery.

In the preferred embodiment the internal bore of the sleeve component is inwardly tapered. Thus, the taper can be co-axial with the femoral neck although there may be advantages in orienting the axis of the taper slightly more vertical when in position so that it is closer to the average force vector acting on the femoral head during human activity. With this tapered sleeve the interface between the sleeve and the prepared bone is placed in compression to aid in retention and facilitate bone ingrowth. The sleeve bore may be arranged with anti rotation features such as ridges which extend along the length of the sleeve to engage the prepared bone surface and prevent rotation of the sleeve relative to the bone.

It is also an aspect of the invention to provide a kit of ball and sleeve components with not only the usual variety of sizes of ball components etc. to fit the implant to the patient but also with altered geometries to facilitate variation in the offset orientation of the ball component and sleeve relative to the neck axis by the surgeon during surgery. Such a kit may also contain trial components, such as trial ball components that facilitate selection of the ball component to actually be fitted to the patient. It is also an aspect of the invention that the various geometries of the ball components are marked on a non-spherical surface of the ball. Such a marking is visible to the surgeon in selecting and orienting the ball component but does not damage the spherical bearing surface. The various markings and symbols in the ball components may not only identify the particular component, but may also be used to orient the component by indicating features such as offsets or the angular orientation of an axis. This aspect of the invention is particularly important when the orientation of a component feature will not be apparent or measurable when the component is installed.

Another object of the invention is to provide a method for installing the femoral prosthesis described above by appropriately preparing and shaping the femoral head, guiding and fitting the sleeve to a proper orientation on the prepared femoral head, and guiding and fitting the partial ball component onto the sleeve to complete the installation of the prosthesis.

### BRIEF DESCRIPTION OF THE DRAWINGS

FIG. 1 is a cross-sectional side view of the upper portion of a human femur;

FIG. 2 is a cross-sectional side view of an embodiment of the present invention showing a sleeve and ball component installed on a prepared femoral head;

FIG. 3 is a top view of FIG. 2;

FIG. 4 is a perspective view of a sleeve and ball component in accordance with the present invention;

FIG. 5 is a cross-sectional view of the sleeve and ball component of FIG. 4 in assembled configuration;

FIG. 6 is a detailed view of the sleeve of FIG. 5;

FIG. 7 is an alternative configuration of a sleeve;

FIG. 8 is a further alternative configuration of a sleeve;

FIG. 9 is a cross-section view of the ball component of FIG. 5;

FIG. 10 is a perpective view of a sleeve and ball component wherein the ball component has a linear offset with respect to the sleeve axis;

FIG. 11 is a cross-section view of the sleeve and ball component of FIG. 10;

FIG. 12 is a cross-section view of the ball component of FIG. 11;

FIG. 13 is a prospective view of the ball component of FIG. 12;

FIG. 14 is a bottom view of the ball component of FIG. 12;

FIG. 15 shows an assembled sleeve and ball component where the sleeve cavity axis has an angular offset with respect to the sleeve outer surface axis and the ball component bore axis has an angular offset with respect to an axis defined by the sphere center and a distal plane;

FIG. 16 shows a perspective view of the ball component of FIG. 15; and

FIG. 17 shows in cross-sectional view an embodiment of the present invention as in FIG. 1 where the femoral head is prepared in an upwardly directed secondary axis B'.

### DETAILED DESCRIPTION

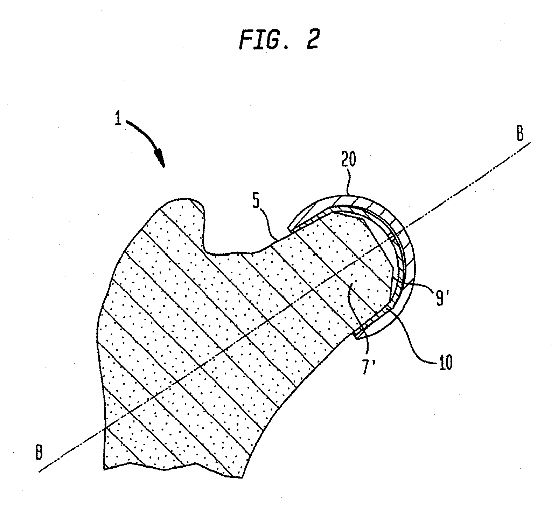

As shown in FIG. 2, a proximal femur as depicted in FIG. 1 has been surgically prepared for the implantation of a femoral hip resurfacing prosthesis. The preparation consists of a re-shaping of the femoral head 7, in this instance, as a surface of revolution about the femoral neck axis B-B. The femoral head 7 has been re-shaped by known surgical techniques as a prepared femoral head 7', such that the femoral head surface 9 has been removed, creating a prepared femoral head surface 9'. Arranged in close contact with the prepared femoral head surface 9', is a sleeve 10. In turn, a ball component 20 is fitted over the sleeve 10. In this manner, a modular prosthesis comprising the sleeve and ball is emplaced on the prepared femoral head with various embodiments and advantages as will be further described.

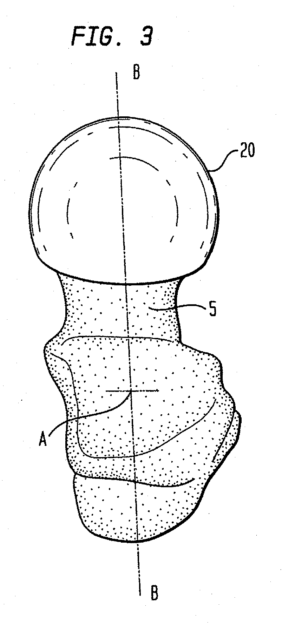

FIG. 3 depicts a top view of the prosthesis of FIG. 2 fitted on a prepared femoral head. The projection of the femoral shaft axis A-A, depicted in FIG. 1, is shown on the upper surface of the greater trochanter. The femoral neck axis B-B passes approximately through the center of the prepared femoral head and, in this instance, the center of the ball component 20 and also approximately through the center of the femoral neck 5.

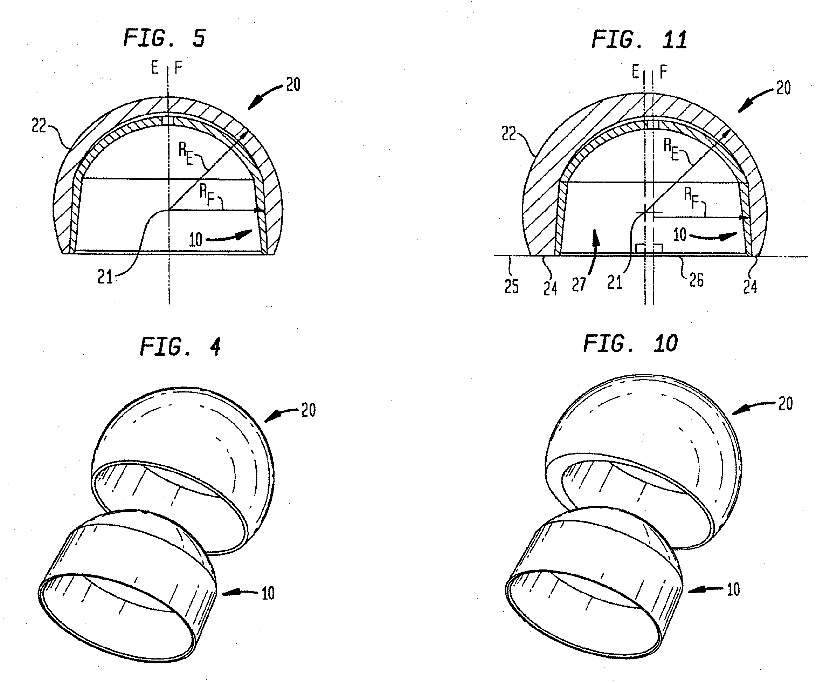

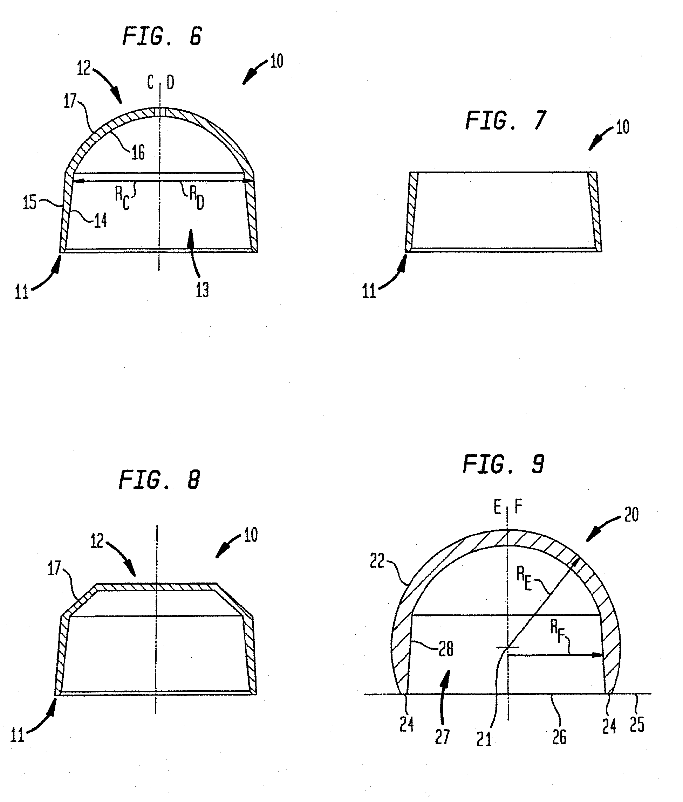

FIG. 4 shows in an exploded perspective view the prosthesis of FIG. 2. It can be seen that the sleeve component 10 which is fitted on the prepared femoral head 7', fits closely inside at least a portion of the ball component 20. It can further be seen in FIG. 6 that the sleeve 10 is generally a solid of revolution about a central axis having a sleeve cavity 13 which is configured to interface with the prepared femoral head surface 9'. The sleeve has a distal portion 11 and a proximal portion 12. In this instance, the distal portion is in the configuration of a hollow truncated cone, having an inner surface 14 and an outer surface 15. For a given position along the central axis, the inner surface 14 can be characterized by a radius Rc and the outer surface can be characterized by a radius Rd. The sleeve inner surface 14 is a surface of revolution characterized by a radius from the central axis, Rc. Rc can characterize a tapered or other variable surface of revolution and therefore is not to be taken as a constant radius for a given position along the axis C. For example, as shown in FIG. 6, Rc will be shorter in the proximal region and longer in the distal region of the distal inner surface 14 in accordance with the tapered geometry shown. In the same manner, the distal outer surface 15 of the sleeve is a surface of revolution having radii Rd. The surface of revolution 14 characterized by Rc defines the central axis C and the surface of revolution 15 characterized by Rd defines a central axis D. As depicted in FIG. 6, C and D are coincident. Thus, the axis C is defined by the sleeve inner surface 14 of the sleeve cavity 13 and is referred to here as the cavity axis. The axis Rd is defined by the sleeve outer surface 15 and is referred to as the sleeve axis. It is not necessary that the cavity axis C and the sleeve axis D be coincident. As will be seen later the axes can be offset from each other linearly, rotationally, or in a combination offset.

While shown here as a truncated cone with two tapering surfaces 14 and 15, either of surfaces 14 and 15 can define a hollow cylinder or other surfaces such as an ogive or any parabolic surface capable of being fit over a matched prepared femoral head surface 9'. The proximal portion 12 can be a different shape of revolution about the central axis or, as shown in FIG. 7, may not even be present. When present, the proximal portion may be closely configured to the prepared femoral head surface 9' or may have clearance from the prepared femoral head surface. The proximal portion of the sleeve 12 has an inner surface 16 and an outer surface 17. As shown in FIG. 6, the proximal portion of the sleeve 12 can be in the configuration of a spherical dome, or alternatively, can be other configurations such as the chamfered configuration shown in FIG. 8. While typically the outer surface 15 of the distal portion of the sleeve 11 fits tightly with the matching inner surface 28 of the ball component 20, it can be seen, as in FIG. 5, that the proximal portion 12 can have clearance with respect to the cavity of the ball component 20.

The sleeve 10 may be a solid structure, or it may have a porous inner surface at 14 that is integrated with or attached to a solid outer layer or the sleeve may be porous throughout. When a taper lock type of retention of the ball component 20 of the sleeve 10 is used as depicted in FIG. 5, it is important that the sleeve be sufficiently rigid in its overall structure when implanted to retain its taper lock characteristic. The porous structure on the inner surface of the sleeve 14, is of a configuration to promote bone ingrowth of the prepared femoral head surface 7' into the mating surface of the sleeve 10, as is known in the art. The thickness of the porous structure may be variable over the inner surface of the sleeve and it may have a gradient of porosity and other characteristics, generally being more porous at the inner surface 14 and dense at the outer surface 15. The characteristics and fabrication of such tissue ingrowth surfaces, either porous or a textured solid, are known in the art, for example technologies such as titanium foam and selective laser sintering can be used to create porous structures and gradient porous structures with variations of pore characteristics such as the pore size, pore interconnectivity and porosity. The porous and solid portions of the sleeve 10 are preferably made from biocompatible metals, such as titanium, titanium alloys, cobalt chrome alloy, stainless steel, tantalum and niobium. The most preferred metals are titanium and titanium alloys. Optionally, additional bioactive materials can be incorporated in the porous sleeve inner surface 14 as are well known in the art such a bone morphogenic protein to promote bone ingrowth, calcium hydroxyapatite and tricalcium-phosphate, to promote bone adhesion to the porous sleeve inner surface, and antibiotics, to reduce the potential for infections and promote healing. As an alternative to retention by bone ingrowth, bone cement may also be used to retain the sleeve.

Different methods may be used to transition the porosity characteristics from a porous sleeve inner surface 14 to an outer surface 15 that is solid or substantially solid. For example, a first region adjacent the sleeve outer surface 15 may be relatively dense, having a porosity in the range from 0% to 50% and the second porosity region adjacent to the porous inner surface 14 may have a relatively greater porosity in the range from 20% to 90%. In the instance of overlapping porosity ranges, the porosity will generally be less in the outer porosity region than in the inner porosity region. It is also possible to establish a gradient of porosity throughout the sleeve progressing from a substantially solid outer surface to a porous inner surface. The gradient of porosity through the sleeve layer may be linear, defined in zones as above or by other means. Variations in the porosity characteristics may be used to alter the modulus of elasticity of the sleeve materials and control the rigidity and transitional material properties between porosity zones, differing materials and differing structural load regions. Methods of achieving distributions of porosity are also discussed in co-owned application No. 10/317,229 entitled "Gradient Porous Implant".

Turning to FIG. 9, the femoral ball component 20 as shown in FIGS. 4 and 5 is further detailed. The ball component has a spherical outer surface 22 that serves as the bearing for the implant when assembled with a mating acetabular cup. The radius of the spherical portion of the ball component 20 is designated 22. The ball component 20 has an opening 26 for a bore 27 that has an inner surface 28 having a shape allowing it to closely conform to the distal sleeve outer surface 15.

The ball component 20 is depicted in cross-section in FIGS. 5, 11 and 12. The hemispherical bearing surface 22 defines a center 21 having a radius Re, the distal plane 25 defines the extent of the surface and also a distal surface 24. The body of the ball component 20 is preferably made of a metallic material similar to those described for the sleeve 10 with the exception that the material is typically solid throughout and has a suitable hardness and durability to provide a bearing surface or substrate. For durability and bearing performance, the ball component 20 may be coated or have a surface layer of ceramic material, or may be entirely composed of a ceramic.

Unlike the hemispherical outer surface 22, the distal surface 24 does not function as a bearing, and does not require the fine finish, hardness and careful handling typically required by an implant bearing surface. Distal surface 24 is depicted in the various figures as co-planar with the distal plane 25. It is to be understood that this is for convenience and clarity of depiction. The distal surface 24 may in fact vary from the distal plane 25. For example, the variation could be defined as the height variation of surface 24 with respect to the distal plane 25 as an angle theta is rotated about the polar axis E. In the instance where the distal surface 24 is not co-planar with the distal plane 25, the distal surface can define a distal plane by setting the distal surface 24 on a known planar surface and defining the plane 25 by the three contact points of the known surface with the distal surface 24 or by other methods as are known in the art.

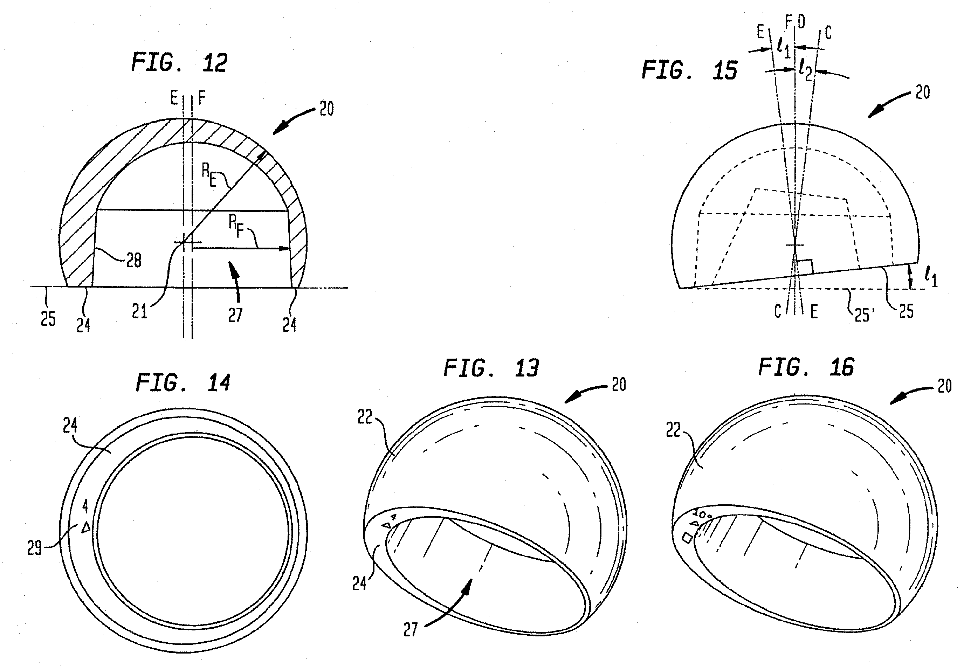

A polar axis E of the ball component 20 as shown in FIGS. 5, 11 and 12 is defined by a line passing through the center 21 of the ball component 20 and perpendicular to the distal plane 25. The bore 27 is a surface of revolution defined by an axis F and radii Rf perpendicular to central axis F. As depicted in FIG. 5, bore 27 can be perpendicular to the distal plane 25 and centered on the center 21 in which case axes E and F are coincident. However, it is an important aspect of the invention that the axes E and F need not be coincident. As shown in FIG. 11 and related figures, the bore is linearly offset with respect to the ball component center 21 such that axis F is to the right of axis E. As will be further discussed, the axis E can also have an angular offset from the axis F depending on the orientation of the bore axis and the distal plane 25.

One difficulty encountered by a surgeon in using spherical components with linear or angular offsets is that the offsets may be difficult to perceive, even in the uninstalled component, and become virtually impossible to discern once the component is installed. For this reason, markings and symbols 29 are provided on the distal surface 24. Comparing Fig. 5 with Fig. 11 it can be seen that an offset ball component provides a relatively larger distal surface 24 suitable for marking. The location of such a marking indicating an offset on the distal surface 24 is important because the bearing surface 22 is unavailable for such a marking as a marking would interfere with its function as a bearing. The markings can show the magnitude and direction or orientation of a linear or angular offset or a combination of these offsets. Thus, in the instance shown in FIGS. 13 and 14, a linear offset of, for instance 4mm, is indicated and the triangle symbol shows the direction of the offset. If desired, a tooling feature such as a hole or holes in the distal surface 24 may also be used to indicate the orientation and magnitude of the offset externally by using a fixturing or indicating tool. Such an indicating tool may be integrated in a tool for holding and impacting the ball component 20 on the sleeve 10.

FIG. 15 shows a ball component 20 with an angular offset. A sleeve 10 with an angular offset is also drawn in phantom. As shown in FIG. 15, the cavity axis F of the ball component is perpendicular with a line indicating a virtual distal plane 25' and is, as previously defined, an axis of symmetry for the bore inner surface 28. The actual distal plane 25 is shown at an angle phi 1 with respect to the virtual plane 25 and indicates the actual machined dimension of the distal surface 24. Consequently, the axis E through the center of the hemispherical surface 22 and perpendicular to the distal plane 25 also has an angular offset phi 1. As mentioned, the sleeve can also incorporate an angular offset feature wherein the axis C defined by the sleeve cavity is at an angle phi 2 to the axis D, defined by the sleeve outer surface. Typically, when assembled, the bore axis F of the ball component will be coincident with the sleeve axis B of the sleeve as shown in FIG. 15 because of the use of a taper lock type fit between the components.

It is also possible to vary the positions of the sleeve 10 and ball component 20 along any of the axes C, D, E and F by varying the relationship of the interface dimensions interface to create a translational offset. For example, in the instance of a conical interface, a relative decrease of Rc with respect to a mating surface of the prepared femoral head 7' will shift the sleeve 10 and the ball component 20 in in the proximal direction along axis C. Similarly, the ball component 20 can be shifted along axes D, E and F by adjusting the various dimensions of the sleeve or sleeve/ball component interface.

It will be understood by a person skilled in the arts that angular, linear and translational offsets can be combined in either or both of the ball component and the sleeve to achieve desired geometrical relationships between the prepared femoral head 7' and the objective position in space of the spherical surface 22. In such instances, more complex markings 29 as indicated by the addition of a square symbol in FIG. 16 may be required and it will also be appreciated that such markings could also be applied to the distal rim or another visible portion of the sleeve 10 knowing that in the instance of the sleeve 10 it is permissible to mark the sleeve on either of the outer surfaces 15 or 17. It will also be understood that the various offsets require a larger radius Re, or a smaller prepared femoral surface 7', than an implant where the ball component surface 22 is centered on the prepared femoral surface.

While the bone ingrowth porous surface described as a preferred embodiment of the sleeve 10 and the taper lock fitting between the sleeve 10 and the ball component 20 are sufficient to prevent rotation of either the sleeve and ball components of the implant, it may be desirable to use ribs or eccentric features such as a key and keyways to insure that rotation does not take place and to provide an indexed orientation between the various components. For example, the interface between the sleeve 10 and the ball component 20 could take the form of a tapered spline rather than a taper lock depending solely on friction. Likewise, the interior surface of the sleeve 14 can have ribs oriented in line with the cavity axis C to provide a mechanical anti-rotation feature and a rotational orientation feature. If desired, the preparation of the femoral head can also include mating features to the sleeve anti-rotation features

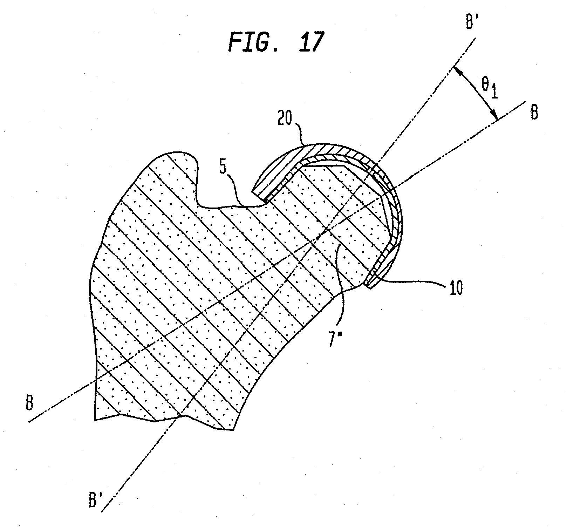

In addition to the angular offsets that can be achieved with the sleeve or the ball component, it is also possible to increase the angular offset by preparing the femoral head 7' on an axis varying from the femoral neck axis B-B. Such an offset preparation axis B'-B' is depicted in FIG. 17. While the axis may be offset in different directions, for example in the posterior direction, the axis shown is upward in direction. Such a configuration is believed to better place the trabeculae of the femoral neck 5 in compression along the interface with the sleeve inner surface 14 and may provide an improved load path into the prepared femoral head 7'. In this instance, the offset of the sleeve, or more preferably the ball component may be used to further increase or decrease the net angular offset of the outer surface 22 of the ball component with respect to the neck axis BB.

The modular components of an implant according to the embodiments of the invention described above are particularly well suited for inclusion in a kit that can be used by a surgeon to evaluate and construct an implant specifically tailored to the patient's autonomy and dimensions. Such a kit of ball and sleeve components can include not only the usual variety of sizes of ball components etc. to fit the implant to the patient but also include components with altered geometries to facilitate variation in the offset orientation of the ball component and sleeve relative to the neck axis, as described above, by the surgeon during surgery. Importantly, the sleeve, once installed on the prepared femoral head, provides a reliable mechanical datum to provide adjustment and optimization of the position of the bearing surface as facilitated by the kit components.

The kit may also contain trial components, such as trial ball components that facilitate selection of the ball component to actually be fitted to the patient by duplicating various aspects of the ball components geometry. The trial components may include features that ease trial fitting but are not possible on an actual component. These features can include transparent components to allow visualization of otherwise obscured regions, external markings and orienting guides on the trial ball surface and tooling points on the trial ball surface. Features can also be incorporated to ease trial fitting, such as taper lock type features that provide accurate positioning, but do not readily lock so as to allow trial rotation of an offset component and ease of removal of the trial component.

As discussed above it is also an aspect of the invention that the various geometries of the ball components are marked on a non-spherical surface of the ball. It will be apparent that given the variety of ball components in a kit and the need for orientating offset components during fitting, the markings and symbols on the distal surface of the ball components may not only serve to identify the particular components, but may also be used to orient the component by indicating the direction and magnitude of features such as offsets or the angular orientation of an axis.

Another object of the invention is to provide a method for installing the femoral prosthesis described above by appropriately preparing and shaping the femoral head, guiding and impacting the sleeve to a proper orientation on the prepared femoral head, and guiding and orienting the ball component onto the sleeve to complete the installation of the prosthesis. The various aspects of the kit described above may also be used during the surgical procedure. It will also be appreciated that even after fitting the actual ball component to the sleeve, the ball component can be removed and a ball component with a different offset or diameter can be used to improve the position of the bearing surface.

As an example of the method of installing a femoral prosthesis to a femoral ball or head, the outer surface of femoral head is first reamed and otherwise shaped to a predetermined configuration to match the shape of the sleeve and create a prepared femoral head having the desired head axis orientation; then a sleeve according to the embodiments of the invention discussed above is fitted on the prepared femoral head. If the sleeve is of the offset type, it is fitted in a desired orientation to properly position the offset. A ball component according to the embodiments of the invention discussed above is then fitted to the sleeve and locked in position. If the ball component is of the linear or angular offset type, it is fitted in a desired orientation to properly position the offset.

It will also be appreciated that in a revision surgery, the original ball component can be removed and a new ball component can be fitted to the original sleeve to replace a ball component or to revise the position of the bearing surface.

Unless stated to the contrary, any use of the words such as "including," "containing," "comprising," "having" and the like, means "including without limitation" and shall not be construed to limit any general statement that it follows to the specific or similar items or matters immediately following it.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A femoral prosthesis adapted to be installed to a prepared natural femoral head, the prepared head having an outer surface and a head axis defined by symmetry with said outer surface, comprising:
a partially cone shaped sleeve having an open distal end and a proximal end, said sleeve having a conical outer surface between said distal end and said proximal end, said conical outer surface defining a sleeve axis, said sleeve having a cavity defining the opening in said open distal end, said cavity having a porous inner surface adapted to engage said outer surface of said prepared natural femoral head and a cavity axis defined by symmetry with said cavity inner surface and adapted to be substantially coincident with said head axis, said sleeve having a larger outer diameter at said distal end and a smaller outer diameter at said proximal end;
a partial ball component capable of conforming to an acetabular socket, said component having a partially spherical outer surface defining a center and a radius of said component, said component having a distal surface defining a distal plane with an opening in said distal surface, a polar axis defined by a line perpendicular to said distal plane and intersecting the center of said component, said opening being formed by a blind bore in said distal surface, said bore having a conical inner surface adapted to fit said conical outer surface of said sleeve and a bore axis defined by symmetry with said conical inner surface.

2. The femoral prosthesis as set forth in claim 1 wherein said bore axis does not intersect said ball component center.

3. The femoral prosthesis as set forth in claim 2 wherein a location feature on the outside of said ball component indicates the spatial relationship between said bore axis and said ball component center.

4. The femoral prosthesis as set forth in claim 3 wherein a location feature on said distal surface indicates the spatial relationship between said bore axis and said ball component center.

5. The femoral prosthesis as set forth in claim 1 wherein said bore axis forms a non-zero angle ϕ₁ with said polar axis.

6. The femoral prosthesis as set forth in claim 5 wherein a location feature on the outside of said component indicates the spatial relationship between said bore axis and said polar axis.

7. The femoral prosthesis as set forth in claim 6 wherein a location feature on said distal surface indicates the spatial relationship between said bore axis and said polar axis.

8. The femoral prosthesis as set forth in claim 1 wherein said cavity axis forms a non-zero angle ϕ₂ with said sleeve axis.

9. The femoral prosthesis as set forth in claim 8 wherein a location feature on the outside of said sleeve indicates the spatial relationship between said cavity axis and said sleeve axis.

10. The femoral prosthesis as set forth in claim 9 wherein a location feature on said distal end of said sleeve corresponds to the spatial relationship between said cavity axis and said sleeve axis.

11. The femoral prosthesis as set forth in claim 1 wherein said cavity axis is offset and parallel to said sleeve axis.

12. The femoral prosthesis as set forth in claim 11 wherein a location feature on the outside of said sleeve indicates the spatial relationship between said cavity axis and said sleeve axis.

13. The femoral prosthesis as set forth in claim 12 wherein a location feature on said proximal end of said sleeve indicates the spatial relationship between said cavity axis and said sleeve axis.

14. The femoral prosthesis as set forth in claim 1 wherein said bore axis is not perpendicular to said distal plane.

15. The femoral prosthesis as set forth in claim 1 wherein said partially spherical outer surface comprises a proximal hemisphere opposite said opening, said polar axis defining an equator of said surface and said proximal hemisphere.

16. The femoral prosthesis according to claim 15 wherein said partially spherical outer surface extends beyond said proximal hemisphere.

17. The femoral prosthesis according to claim 16 wherein said partially spherical outer surface extends beyond said proximal hemisphere to differing extents depending on an angle of rotation θ about said polar axis.

18. The femoral prosthesis as set forth in claim 1 wherein said proximal end of said sleeve comprises a dome.

19. The femoral prosthesis as set forth in claim 1 wherein said proximal end of said sleeve comprises a chamfered surface.

20. The femoral prosthesis as set forth in claim 1 wherein said porous inner surface of said sleeve comprises a conical inner surface and a proximal inner surface.

21. The femoral prosthesis as set forth in claim 1 wherein said proximal inner surface of comprises a dome.

22. The femoral prosthesis as set forth in claim 1 wherein said proximal inner surface of comprises a chamfered surface.

23. The femoral prosthesis as set forth in claim 1 wherein said proximal end of said sleeve has an aperture centered at the intersection of said cavity axis with said proximal end.

24. The femoral prosthesis as set forth in claim 1 wherein the outside of said sleeve is solid metal.

25. The femoral prosthesis as set forth in claim 22 wherein the thickness between the outside of said sleeve and said porous inner surface is a zone of gradient porosity where the porosity decreases through said layer along a gradient from said porous inner surface to said solid metal outer surface.

26. The femoral prosthesis as set forth in claim 23 wherein the rate of decrease of the porosity through said sleeve layer is linear.

27. The femoral prosthesis as set forth in claim 1 wherein the porosity in a first porosity region adjacent the outside of said sleeve has a first porosity in the range from 0% to 50% and in a second porosity region adjacent said porous inner surface has a second porosity in the range from 20% to 90%.

28. The femoral prosthesis as set forth in claim 1, wherein said sleeve is substantially composed of a metal selected from the group of titanium, titanium alloys, cobalt chrome alloys, niobium and tantalum.

29. The femoral prosthesis as set forth in claim 26, wherein said porous inner surface is coated with a material selected from the group of bone morphogenic protein, calcium hydroxyapatite, tri-calcium phosphate, and antibiotics.

30. The femoral prosthesis as set forth in claim 1, wherein said ball component is substantially composed of a metal selected from the group of titanium, titanium alloys, cobalt chrome alloys, niobium and tantalum.

31. The femoral prosthesis as set forth in claim 27, wherein said ball component partially spherical outer surface is coated with a ceramic.

32. A kit for use in installing a femoral prosthesis on a prepared natural femoral head, the prepared head having an outer surface and a head axis defined by symmetry with said outer surface, said kit comprising:
a partially cone shaped sleeve having an open distal end and a proximal end, said sleeve having a conical outer surface between said distal end and said proximal end, said conical outer surface defining a sleeve axis, said sleeve having a cavity defining the opening in said open distal end, said cavity having a porous inner surface adapted to engage said outer surface of said prepared natural femoral head and a cavity axis defined by symmetry with said cavity inner surface and adapted to be substantially coincident with said head axis, said sleeve having a larger outer diameter at said distal end and a smaller outer diameter at said proximal end;
a first partial ball component capable of conforming to an acetabular socket, said first component having a partially spherical outer surface defining a center and a radius r₁ of said component, said component having a distal surface defining a distal plane with an opening in said distal surface and a polar axis defined by a line perpendicular to said distal plane and intersecting the center of said component, said bore having a conical inner surface adapted to fit said conical outer surface of said sleeve and a bore axis defined by symmetry with said conical inner surface, the geometric relationship of said bore axis and said polar axis defining a first axes relationship;
a second partial ball component capable of conforming to an acetabular socket, said second component having a partially spherical outer surface defining a center and a radius r₂ of said component, said component having a distal surface defining a distal plane and an opening in said distal surface and a polar axis defined by a line perpendicular to said distal plane and intersecting the center of said component, said opening being formed by a blind bore in said distal surface, said bore having a conical inner surface adapted to fit said conical outer surface of said sleeve and a bore axis defined by symmetry with said conical inner surface, the geometric relationship of said bore axis and said polar axis defining a second axes relationship, said second component having a varying geometry from the geometry of said first component created by varying one or more of said radius r₂ or said second axes relationship from said first axes relationship;
a first trial component corresponding to the geometry of said first partial ball component;
a second trial component corresponding to the geometry of said second partial ball component.

33. The kit as set forth in claim 31 wherein a location feature on the outside of any of said first ball component, said second ball component, said first trial ball component or second ball component indicates the geometric relationship between a corresponding bore axis and polar axis.

34. The kit as set forth in claim 31 wherein a location feature on said distal surface of any of said first ball component or said second ball component, indicates the geometric relationship between a corresponding bore axis and polar axis.

35. A kit for use in installing a femoral prosthesis on a prepared natural femoral head, the prepared head having an outer surface and a head axis defined by symmetry with said outer surface, said kit comprising:
a first partially cone shaped sleeve having an open distal end and a proximal end, said sleeve having a conical outer surface between said distal end and said proximal end, said conical outer surface defining a sleeve axis, said sleeve having a cavity defining the opening in said open distal end, said cavity having a porous inner surface adapted to engage said outer surface of said prepared natural femoral head and a cavity axis defined by symmetry with said cavity inner surface and adapted to be substantially coincident with said head axis, said sleeve having a larger outer diameter at said distal end and a smaller outer diameter at said proximal end, the geometric relationship of said cavity axis and said sleeve axis defining a first axes relationship;
a second partially cone shaped sleeve having an open distal end and a proximal end, said sleeve having a conical outer surface between said distal end and said proximal end, said conical outer surface defining a sleeve axis, said sleeve having a cavity defining the opening in said open distal end, said cavity having a porous inner surface adapted to engage said outer surface of said prepared natural femoral head and a cavity axis defined by symmetry with said cavity inner surface and adapted to be substantially coincident with said head axis, said sleeve having a larger outer diameter at said distal end and a smaller outer diameter at said proximal end, the geometric relationship of said cavity axis and said sleeve axis defining a second axes relationship, said second sleeve having a varying geometry from the geometry of said first sleeve created by varying said second axes relationship from said first axes relationship;
a partial ball component capable of conforming to an acetabular socket, said first component having a partially spherical outer surface defining a center and a radius r₁ of said component, said component having a distal surface defining a distal plane with an opening in said distal surface and a polar axis defined by a line perpendicular to said distal plane and intersecting the center of said component, said bore having a conical inner surface adapted to fit said conical outer surface of said sleeve and a bore axis defined by symmetry with said conical inner surface.
a first trial sleeve corresponding to the geometry of said first partially cone shaped sleeve;
a second trial sleeve corresponding to the geometry of said second partially cone shaped sleeve.
